# FASCICULE DE BREVET EUROPEEN

(11) **EP 2 894 978 B1**
(45) Date de publication et mention de la délivrance du brevet: **18.07.2018**
(21) Numéro de dépôt: 13773633.6
(22) Date de dépôt: 10.09.2013
(51) Int. Cl.: A01N 25/28, A61K 8/11, A61K 9/50, C09B 67/02, C11D 3/50

(54) **PROCEDE ALTERNATIF DE MICROENCAPSULATION DE PRINCIPES ACTIFS**
ALTERNATIVES VERFAHREN ZUR MIKROVERKAPSELUNG VON WIRKSTOFFEN
ALTERNATIVE METHOD FOR MICROENCAPSULATION OF ACTIVE INGREDIENTS

(30) Priorité: 11.09.2012 FR 1258503
(43) Date de publication de la demande: 22.07.2015
(73) Titulaire: Creathes, 90000 Belfort (FR)
(72) Inventeur: HUILIER, Hervé, F-70400 Errevet (FR)
(74) Mandataire: Pontet Allano & Associes
(86) Numéro de dépôt international: PCT/EP2013/068760
(87) Numéro de publication internationale: WO 2014/041001

(56) Documents cités:
- EP-A1- 2 295 044
- WO-A1-99/39700
- WO-A1-2012/109755
- US-A1- 2006 024 248
- GIANNOLA L I ET AL: "CARNAURA WAX MICROSPHERES LOADED WITH VALPROIC ACID: PREPARATION AND EVALUATION OF DRUG RELEASE", DRUG DEVELOPMENT AND INDUSTRIAL PHARMACY, NEW YORK, NY, US, vol. 21, no. 13, 1 janvier 1995 (1995-01-01), pages 1563-1572, XP000677299, ISSN: 0363-9045, DOI: 10.3109/03639049509069246

## Description

### Domaine technique

La présente invention se rapporte à un procédé de microencapsulation permettant de répondre à de nombreuses problématiques pour la cosmétique et l'alimentaire notamment, mais aussi pour l'agrochimie ou la détergence.

La présente invention permet ainsi de fabriquer une suspension de microcapsules solides dans un milieu liquide grâce à un procédé nécessitant deux étapes majeures : une étape d'émulsification à chaud et une étape de cristallisation à froid. Ces capsules sont utiles, par exemples, (i) pour fabriquer une composition cosmétique choisie parmi les crèmes, les lotions, les gels et les émulsions, ou (ii) pour fabriquer des tissus ou papiers imprégnés, voire (iii) pour fabriquer des produits ménagers, phytosanitaires ou agrochimiques.

### Etat de la technique

Depuis quelques années, les applications de la microencapsulation ne cessent de croître notamment dans l'alimentaire et la cosmétique spécialement pour des applications dites « long lasting », de libération intelligente et de protection des principes actifs. L'effet « long lasting » consiste en un effet durable dans le temps : les capsules, qui contiennent un principe actif, le libèrent petit à petit au cours du temps. La microencapsulation a donc dû s'adapter à ces applications et aux secteurs visés donnant ainsi lieu à des technologies spécifiques aussi bien chimiques que physiques.

En outre, nombreux sont désormais les secteurs qui attachent une importance toute particulière dans le choix des matières premières non seulement du point de vue de leur innocuité mais également de leur impact sur l'environnement. C'est ainsi qu'une tendance, en constante croissance, se dirige vers les procédés verts et la microencapsulation en fait partie. De nouvelles technologies plus « propres », respectueuses de l'environnement et employant des matériaux verts, sont donc de plus en plus recherchées.

En parallèle de l'image des produits, ce sont également des questions de santé publique qui sont soulevées. A l'image de la cosmétique, nombre de secteurs durcissent les réglementations et les listes noires de matières à proscrire. C'est ainsi que, de plus en plus, la microencapsulation doit se tourner vers des procédés sans réactions chimiques, avec des matières issues du monde végétal et présentant une excellente biocompatibilité.

Actuellement, la microencapsulation est essentiellement issue de travaux menés pour le papier, le domaine pharmaceutique et l'agroalimentaire avec des procédés employant des matériaux de synthèse, des procédés lourds comprenant des réactions chimiques, pas toujours adaptés à d'autres secteurs de part leurs caractéristiques et les matériaux employés.

Le brevet EP346034B1 divulgue un procédé de préparation de particules de matériau actif comprenant :
- la dispersion dudit matériau actif dans une cire fondue à 55°C, par exemple, pour former une dispersion de matériau actif dans la cire ;
- l'addition de ladite dispersion à l'eau, maintenue par exemple à 60°C, et qui contient au moins un tensioactif et l'émulsion de la dispersion de matériau actif dans la cire en moins de 4 minutes pour y former des capsules ;
- refroidissement immédiat par adjonction d'eau froide à 10°C, par exemple, après formation desdites capsules et
- récupération desdites capsules refroidies dans ladite eau.

La vitesse d'émulsification est de 300 à 1200 tpm. Les capsules obtenues avec ce procédé ont un coeur de matériau actif et un enrobage à base d'un mélange de cires dure et molle ayant un point de fusion de 50 à 80°C et un diamètre de 500 à 1200 micromètres avec une taille moyenne d'environ 700 micromètres.

La demande WO2009/046930 divulgue un procédé de fabrication de microcapsules de diamètre moyen de 0,1 à 5000 micromètres dans lequel des cires et des actifs lipophiles sont mélangés à une température au-dessus de la température de fusion des cires, par exemple à 78°C, de façon à former une première phase lipophile, ladite phase étant ensuite mise en suspension dans une solution aqueuse contenant un agent de suspension hydrophile et optionnellement d'un tensioactif permettant une émulsion huile-dans-eau. Le mélange ainsi obtenu est ensuite refroidi brutalement en dessous de la température de fusion des cires, par exemple à 5°C, de manière à former des capsules qui sont séparées de la suspension.

La demande WO 99/39700 décrit un procédé de préparation de nanoparticules de diamètre compris entre 50 et 500 nm, comprenant les étapes suivantes :
- chauffer ensemble au moins une substance lipidique, au moins une substance amphiphile et un principe actif à une température au-dessus de la température de fusion d'au moins l'une de ces substances,
- ajouter une solution aqueuse dans ladite composition pour former une microémulsion,
- disperser ladite microémulsion dans l'eau ou une solution aqueuse de +1°C à +10°C pour former une suspension de nanoparticule.

Le document US 2006/024248 décrit également un procédé de préparation de nanoparticules, permettant de produire les nanoparticules ayant un diamètre moyen de 10 nm à 1000 nm. Ledit procédé comprend: (i) le chauffage d'une phase grasse (phase B) comprenant une substance lipidique et un principe actif, à une température supérieure à la température de fusion de la substance lipidique; (ii) le chauffage d'une phase aqueuse (phase A) comprenant au moins un tensio-actif; (iii) l'incorporation de ladite phase grasse dans ladite première phase aqueuse pour former une émulsion huile-dans-eau, et (iv) l'addition de ladite émulsion dans une seconde phase aqueuse (phase C), la température de ladite seconde phase étant inférieure à la température de fusion de ladite substance lipidique et conduisant à la formation de nanoparticules.

La demande de EP2295044 concerne un procédé de production de capsules stables, qui comprend (a) le mélange homogène d'une substance à encapsuler dans au moins une substance de type cire, la dispersion du mélange de l'étape (a) dans une solution aqueuse en utilisant au moins un dispersant, le refroidissement et la dilution de la dispersion de l'étape (b). Les capsules ont un diamètre maximum de moins de 1 micromètre, préférablement de moins de 0,25 micromètres.

Le brevet US-3,856,699 est relatif à un procédé de fabrication de capsules de type « core-shell » ayant une membrane externe ou « shell» en matériau cireux. L'actif à encapsuler est dispersé dans une phase grasse, liquide à chaud, dans un premier réacteur dont la température est supérieure à la température de fusion de ladite phase grasse. Une phase aqueuse est disposée dans un second réacteur cylindrique séparé en deux compartiments. Dans la partie supérieure A de ce réacteur, la phase aqueuse est agitée et chauffée à une température supérieure à la température de fusion de la phase grasse. Dans la partie inférieure B du réacteur, une seconde phase aqueuse, de même nature que la précédente, est refroidie à une température inférieure à la température de fusion de la phase grasse. L'ensemble de la phase grasse (matrice + actif) est versé dans le compartiment A, c'est-à-dire dans la phase aqueuse chaude, et agité pour contrôler la taille de l'émulsion formée. Puis, le procédé permet aux gouttes de phase grasse de tomber par gravité dans la partie inférieure B, froide et non agitée. On a donc une cristallisation de la matrice en parois qui forment des capsules et tombent au fond du récipient B. Les capsules obtenues ont une taille entre 20 et 500 micromètres, contrôlée par l'agitation de la phase aqueuse du compartiment A. Cependant, le procédé décrit dans ce brevet ne peut pas être utilisé pour fabriquer des microcapsules dont la densité serait inférieure à celle de la phase aqueuse dans laquelle elles se formeraient.

Les procédés simples et « verts » sont essentiellement des procédés physiques tels que par exemple (i) le « spray cooling » ou procédé de congélation de gouttes et (ii) le « spray drying » ou procédé de séchage par atomisation. Mais, ces procédés ont l'inconvénient d'être très énergivores et les caractéristiques finales des produits obtenus à leur issue ne sont souvent pas compatibles avec les applications qui nécessitent des granulométries inférieures à 50 micromètres.

En effet, un procédé d'encapsulation physique largement répandu est le procédé de congélation de gouttes qui consiste à pulvériser, dans une enceinte, de fines gouttelettes constituées d'une matière fondue telle qu'une cire ou une matière grasse ou autre, ladite matière contenant l'actif solubilisé ou dispersé. La congélation des gouttes se fait par contact avec de l'air frais, l'abaissement de la température des gouttes en dessous du point de fusion provoquant la formation de microcapsules. Cependant, ce procédé d'encapsulation présente de nombreux désavantages définis ci-après:
- la cire fondue doit être de faible viscosité ;
- il est nécessaire de chauffer les tuyauteries afin d'amener la cire fondue à la buse sans congélation en amont voire de sur-chauffer ;
- la concentration en actifs est le plus souvent < 40% pour cause de problèmes de viscosité surtout;
- la répartition granulométrique finale est large ;
- la granulométrie finale est supérieure à 70 micromètres la plupart du temps ;
- il est nécessaire de refroidir une grande quantité d'air.

Une autre méthode, à savoir l'encapsulation par congélation d'une émulsion, dérivée du « spray-cooling » consiste à réaliser une émulsion à chaud contenant une matière grasse fondue dans laquelle est dissous ou dispersé l'actif, cette phase grasse étant ensuite dispersée dans l'eau. La congélation de gouttes se fait alors par abaissement rapide de la température du milieu afin de descendre en dessous du point de fusion de la matrice d'encapsulation. Toutefois, ce procédé d'encapsulation présente également des inconvénients définis ci-après :
- la granulométrie des particules obtenues est soit inférieure à 1 micromètre, soit supérieure à 100 micromètres ;
- le maintien de l'émulsion dans un état stable pendant le changement de la température est très difficile à réaliser ; et
- les dispersions ou « slurries » obtenues sont souvent diluées et ne contiennent que très peu de matière encapsulée (<10%).

Il existe donc un réel besoin de disposer d'un procédé d'encapsulation palliant les défauts, inconvénients et obstacles de l'art antérieur mentionnés ci-dessus. En particulier, il existe un vrai besoin de mettre au point un procédé permettant de maîtriser la granulométrie des microcapsules et de réduire les coûts de fabrication tout en simplifiant les procédés existants.

L'objectif de la présente invention est donc de fournir un procédé simple et économique permettant de fabriquer par congélation, des microcapsules dispersées dans l'eau, et ce sans réaction chimique, lesdites microcapsules ayant une granulométrie ou diamètre des microcapsules pouvant être comprise entre 2 et 80 micromètres, voire encore plus avantageusement comprise entre 5 et 50 micromètres. Lesdites microcapsules peuvent, en outre, relarguer leur contenu, c'est-à-dire le principe actif, par (i) diffusion ou « long lasting », par (ii) érosion, par (iii) pression, ou par (iv) fusion sous l'action de la température.

La solution proposée a vocation d'apporter une alternative à la technologie de congélation de gouttes ou « spray-cooling » à l'échelle industrielle qui permet de façon inattendue d'obtenir une granulométrie des microcapsules que ne permet pas le « spray-cooling ».

La présente invention ne relève donc pas d'une simple utilisation ou adaptation des techniques existantes mais a, au contraire, vocation à créer de nouvelles avancées scientifiques dans le domaine de la microencapsulation, avancées qui n'ont pu être réalisées jusqu'à présent.

La présente invention concerne donc un procédé de fabrication de microcapsules de principe actif, en suspension dans l'eau comprenant les étapes successives suivantes :
(I) Chauffage d'une phase grasse comprenant au moins (i) une cire, naturelle et/ou synthétique, et (ii) au moins un principe actif à encapsuler, à une température réactionnelle supérieure à la température de fusion de ladite cire;
(II) Chauffage d'une première phase aqueuse comprenant au moins un tensio-actif hydrosoluble et au moins un colloïde protecteur, à ladite température réactionnelle, sous agitation mécanique ;
(III) Incorporation de ladite phase grasse dans ladite première phase aqueuse en maintenant la température réactionnelle, sous agitation mécanique, de manière à former une émulsion huile-dans-eau stable;
(IV) Ajout au goutte à goutte de ladite émulsion formée précédemment dans une seconde phase aqueuse comprenant au moins un tensio-actif hydrosoluble ou au moins un colloïde protecteur, la température de ladite seconde phase étant inférieure à la température de fusion de ladite cire, le tout sous agitation mécanique et conduisant à la formation de microcapsules solides, cristallisées contenant ledit principe actif, lesdites microcapsules étant en suspension dans ladite seconde phase aqueuse, lesdites microcapsules ayant un diamètre compris entre 2 et 80 micromètres.
Ainsi conformément à l'invention, la seconde phase aqueuse peut comprendre soit au moins un tensio-actif hydrosoluble et pas de colloïde protecteur, soit au moins un colloïde protecteur et pas de tensio-actif hydrosoluble, soit à la fois un tensio-actif hydrosoluble et un colloïde protecteur.

Les principales difficultés de cette technique de microencapsulation sont de gérer la stabilité de l'émulsion à chaud, d'éviter la séparation de phase entre l'actif et la cire, de gérer la cristallisation des gouttes et la stabilité de l'actif lors de cette phase, de gérer la taille et la forme des microcapsules, de gérer la bonne dispersion des microparticules, de gérer la stabilité de la dispersion en fin de procédé, voire d'obtenir une forme sèche des particules.

L'étape IV d'ajout "goutte à goutte" de l'émulsion chaude dans la seconde phase aqueuse selon l'invention permet une cristallisation brutale de l'émulsion de forte granulométrie sans qu'il y ait déstabilisation du milieu, donc sans coalescence des capsules, sans agglomération des particules, et sans éjection du principe actif ...

Le seul fait que l'émulsion obtenue dans l'étape III soit ajoutée goutte à goutte permet l'encapsulation conformément à l'invention et l'obtention de microcapsules. L'utilisation d'un colloïde protecteur dans la 1ère phase aqueuse combinée avec l'ajout goutte à goutte de l'émulsion permet d'améliorer encore la microencapsulation, alors que la seule présence d'un colloïde protecteur sans ajout goutte à goutte ne permet pas de produire des microcapsules.

Conformément à l'invention, le débit de transfert « goutte à goutte » dans l'étape IV du procédé est fonction de la quantité de dispersion à préparer et l'homme du métier saura le fixer aux vues de ses connaissances générales. Ainsi, pour des lots d'environ 0,5 à 1kg de seconde phase aqueuse, le débit est compris entre 10 à 40 ml/min et pour des lots d'environ 50 kg, le débit peut aller jusqu'à 400 ml/min.

Selon un mode de réalisation avantageux du procédé de l'invention, la phase grasse de la présente invention est constituée par (i) au moins une cire, naturelle et/ou synthétique, et (ii) au moins un principe actif à encapsuler. Cela signifie que ladite phase grasse ne comporte pas de produit amphiphile, de stabilisant, de tensioactif ou de co-tensioactif, ou d'autres additifs qui ne soit pas le principe actif à encapsuler.

Selon un mode de réalisation particulier du procédé, la phase grasse de la présente invention est constituée par (i) une cire naturelle ou synthétique, et (ii) un principe actif à encapsuler.

Selon un mode de réalisation particulier du procédé, la seconde phase aqueuse ne comporte pas de solvant supplémentaire autre que ceux apportés par les ingrédients déjà présents, ce qui permet d'utiliser le moins d'additifs possibles et d'obtenir des microcapsules le plus écologique possible.

Selon un mode de réalisation du procédé, la température réactionnelle est supérieure d'au moins 10°C à la température de fusion de ladite cire.

Selon un mode de réalisation particulier du procédé, la température de la seconde phase est de 10°C à 5°C inférieure à la température de cristallisation de ladite cire.

Selon un mode de réalisation du procédé, la vitesse d'agitation dans l'étape III est de 700 à 6000 trs/min, avantageusement entre 1000 et 4000 trs/min avec le mélangeur IKA Eurostar Control® Vsic 6000.

Selon un mode de réalisation du procédé, le principe actif est liposoluble, lipo-dispersable ou non hydrosoluble.

Selon un mode de réalisation du procédé, le principe actif est choisi parmi les actifs cosmétiques, les parfums, les colorants, les pigments, les répulsifs d'insectes, les molécules volatiles, les poudres, les raticides et les pesticides.

Selon un mode de réalisation du procédé, le colloïde protecteur est choisi parmi la gomme arabique, la gélatine, les dérivés de la cellulose, les alcools polyvinyliques (abrégé PVA), les polyvinylpyrrolidones (abrégé PVP).

Selon un mode de réalisation du procédé, le ou les tensioactif(s) de la première phase aqueuse et/ou de la seconde phase aqueuse est ou sont choisi(s) parmi les tensioactifs non ioniques et les tensioactifs anioniques.

Selon un mode de réalisation du procédé, le ou les tensioactif(s) de la première phase aqueuse et/ou de la seconde phase aqueuse est ou sont choisi(s) parmi les polyoxyéthylènes stearyl éthers, le lauryl sulfonate sodium, les copolymères polyoxyethylene polyoxypropylène (forme AB, ABA, BAB) et les esters de sorbitan. On peut citer comme tensioactif(s) de la première phase aqueuse et/ou de la seconde phase aqueuse le stéareth 21, les Pluronic®, les Tween® et les Span® solubles dans l'eau et capables de former une émulsion huile dans l'eau, et les Synperonic®.

Ces tensioactifs sont bien connus de l'homme du métier.

Ainsi le stéareth 21 est un polyoxyéthylène stearyl éther comprenant 21 unités oxyde d'éthylène par molécule dont le numéro CAS est 9005-00-9. Il est disponible commercialement chez ICI sous la marque Brij 721®.

Pluronic® est la marque sous laquelle BASF commercialise des copolymères polyoxyéthylène polyoxypropylène appelés aussi poloxamères.

Tween® et Span® sont les marques sous lesquelles ICI commercialisent des esters d'acides gras du sorbitan oxyéthylénés. Synperonic® est la marque sous laquelle ICI commercialise des copolymères polyéthylène polypropylène glycol.

Selon un mode de réalisation du procédé, la cire est choisie parmi les cires ayant une température de fusion comprise entre 23 et 85°C, avantageusement comprise entre 55 et 75°C.

Selon un mode de réalisation du procédé:
- le diamètre médian ou d50 des microcapsules obtenues est inférieur à 50 micromètres, plus avantageusement encore compris entre 5 et 50 micromètres, et/ou
- le d10 est supérieur ou égal à 1,4 micromètre, et/ou
- le d90 est compris entre 50 et 90 micromètres,
sachant que le diamètre médian correspond à l'ouverture théorique d'un tamis telle que 50 % des particules, en volume, ont un diamètre supérieur et 50 % un diamètre inférieur au d50, que le d10 correspond à l'ouverture théorique d'un tamis telle que 10 % des particules, en volume, ont un diamètre inférieur au d10, et que le d90 correspond à l'ouverture théorique d'un tamis telle que 90 % des particules, en volume, ont un diamètre inférieur à d90.

Selon un mode de réalisation particulier, le procédé de l'invention a pour objet de fabriquer une dispersion de microcapsules de principe actif contenant au final entre 15 et 45% en masse de microcapsules. Ceci est réalisé en ajoutant au minimum une part et au maximum deux parts de l'émulsion obtenue à l'étape (III) dans une part de la seconde phase aqueuse.

L'invention concerne également l'utilisation de microcapsules obtenues à l'issue du procédé selon l'invention (i) pour fabriquer une composition cosmétique choisie parmi les crèmes, les lotions, les gels et les émulsions, ou (ii) pour fabriquer des tissus ou du papier imprégné(s), ou (iii) pour fabriquer des produits ménagers, phytosanitaires ou agrochimiques.

### Description de l'invention

Par « cire » au sens de la présente invention, on entend un composé lipophile, solide à température ambiante (25°C), à changement d'état solide/liquide réversible, soit par fusion, transition vitreuse ou par ramollissement entre 30°C et 120°C.

Par « liposoluble » au sens de l'invention, on entend « qui est soluble dans les graisses (ex : les huiles, les cires,...) ».

Par « lipo-dispersible » au sens de l'invention, on entend « qui peut être dispersible dans les graisses ».

Par « non hydrosoluble » au sens de l'invention, on entend « qui ne peut pas être soluble dans l'eau ».

Par « principe actif » au sens de l'invention, on entend toute molécule différente de la cire qui nécessite d'être encapsulée pour des questions, par exemple, de conservation, d'utilisation, de manipulation...

Par « colloïde protecteur » au sens de la présente invention, on entend une molécule qui empêche la coalescence des microcapsules.

Par « température ambiante », il faut comprendre une température d'environ 25°C, à pression atmosphérique normale (760 mm de Hg).

Par « température d'usage » au sens de l'invention, on entend la température à laquelle il est envisagé d'utiliser ladite dispersion de microcapsules.

Par exemple, un principe actif à effet anti-moustique contenu dans une dispersion de microcapsules pour cet usage sera choisi parmi les molécules volatiles à environ 40°C, température à laquelle il est envisagé d'utiliser ladite dispersion de microcapsules.

Par « Cx », il faut comprendre une chaine aliphatique, linéaire ou branchée, saturée ou insaturée, comprenant x atomes de carbone.

La phase grasse conforme à l'invention comprend au moins (i) une cire, naturelle ou synthétique, et (ii) au moins un principe actif à encapsuler.

**I/ PHASE GRASSE**

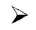 **Cires**

Les cires peuvent être (i) hydrocarbonées, éventuellement substituées, en particulier hydroxylées, (ii) fluorées et/ou (iii) siliconées.

Comme cires conformes à l'invention, on peut citer des cires hydrocarbonées dont le point de fusion est avantageusement supérieur à 35°C, par exemple supérieur à 55°C. De façon générale, le point de fusion de la cire peut être mesuré à l'aide d'un calorimètre à balayage différentiel (D. S. C), par exemple le calorimètre vendu sous la dénomination DSC 30 par la Société METTLER. En tant que cires hydrocarbonées, on peut citer par exemple les cires hydrocarbonées linéaires ou les esters d'acides gras primaires, secondaires ou tertiaires.

Les cires hydrocarbonées peuvent être choisies parmi les cires hydrocarbonées linéaires. On peut citer comme cires hydrocarbonées linéaires : les alcanes linéaires substitués, les alcanes linéaires non substitués, les alcènes linéaires non substitués, les alcènes linéaires substitués. Un composé non substitué est uniquement composé de carbone et d'hydrogène et les substituants ne contiennent pas d'atomes de carbone. Les cires hydrocarbonées linéaires incluent les cires de paraffine linéaires, comme les paraffines S&P® 206, S&P® 173 et S&P® 434 de Strahl & Pitsch.

Les cires hydrocarbonées peuvent être choisies parmi les esters d'acides gras, saturés ou insaturés, ou issues d'acides gras insaturées qui ont été préalablement hydrogénés afin de devenir saturés.

La cire peut ainsi être choisie parmi les esters :
∘ d'acide caprique (en C10), d'acide laurique (en C12), d'acide myristique (en C14), d'acide palmitique (en C16), d'acide stéarique (en C18), d'acide arachidique (en C20), d'acide palmitoléique (en C16), d'acide oléique (en C18), d'acide linoléique (en C18), d'acide linolénique (en C18), d'acide arachidonique (en C20) ; et
∘ d'alcools gras, primaires, secondaires (tel que l'éthylène glycol) ou tertiaires (tel que le glycérol), en C2 à C22, avantageusement en C6 à C18,
et les esters :
∘ d'acide ricinoléique (en C18) ou d'acide ricinoléique hydrogéné ; et
∘ d'alcools gras, primaires, secondaires (tel que l'éthylène glycol) ou tertiaires (tel que le glycérol), en C2 à C22, avantageusement en C6 à C22, mieux encore en C16 à C22 ;
ou leurs mélanges.

Ces esters présentent des points de fusion qui peuvent varier en fonction de l'alcool incorporé de 23°C à 85°C.

On peut citer les esters (i) d'acide myristique et d'éthylène glycol ou de glycérol (ii) d'acide stéarique et d'alcools gras primaires et linéaires en C6 ou d'éthylène glycol ou de glycérol, (iii) les esters d'acide stéarique et d'alcools gras primaires et linéaires en C8, (iv) les esters d'acide stéarique et d'alcools gras primaires et linéaires en C10, (v) les esters d'acide stéarique et d'alcools gras primaires et linéaires en C12, (vi) les esters d'acide stéarique et d'alcools gras primaires et linéaires en C14, (vii) les esters d'acide stéarique et d'alcools gras primaires et linéaires en C16, (viii) les esters d'acide stéarique et d'alcools gras primaires et linéaires en C18, (ix) les esters d'acide ricinoléique, hydrogéné ou non, et d'alcools gras primaires et linéaires en C16 ou d'éthylène glycol ou de glycérol, (x) les esters d'acide ricinoléique, hydrogéné ou non, et d'alcools gras primaires et linéaires en C18 et (xi) les esters d'acide ricinoléique, hydrogéné ou non, et d'alcools gras primaires et linéaires en C22.

Selon un mode de réalisation, la cire est choisie parmi les esters d'acide myristique et d'éthylène glycol ou de glycérol, les esters d'acide stéarique et d'alcools gras primaires et linéaires en C18 ou d'éthylène glycol ou de glycérol, les esters d'acide ricinoléique, hydrogéné ou non, et d'alcools gras primaires et linéaires en C22 ou d'éthylène glycol ou de glycérol, l'huile de palme hydrogénée. Avantageusement, on peut citer le tristéarate de glycérol et le trimyristate de glycérol.

On peut citer l'ester d'acide stéarique et d'alcool gras primaire et linéaire en C6 avec une Tf entre 23 et 28°C, l'ester d'acide stéarique et d'alcool gras primaire et linéaire en C8 avec une Tf entre 27 et 32°C, l'ester d'acide stéarique et d'alcool gras primaire et linéaire en C10 avec une Tf entre 35 et 42°C, l'ester d'acide stéarique et d'alcool gras primaire et linéaire en C12 avec une Tf entre 40 et 45°C, l'ester d'acide stéarique et d'alcool gras primaire et linéaire en C14 avec une Tf entre 45 et 52°C, l'ester d'acide stéarique et d'alcool gras primaire et linéaire en C16 avec une Tf entre 52 et 57°C, l'ester d'acide stéarique et d'alcool gras primaire et linéaire en C18 avec une Tf entre 54 et 60°C, l'esters d'acide ricinoléique hydrogéné et d'alcools gras primaire et linéaire en C16 avec une Tf entre 62 et 66°C, l'esters d'acide ricinoléique hydrogéné et d'alcools gras primaire et linéaire en C18 avec une Tf entre 67 et 71°C, l'esters d'acide ricinoléique hydrogéné et d'alcools gras primaire et linéaire en C22 avec une Tf entre 71 et 75°C.

On pourra citer cires particulièrement avantageuses pour l'invention les cires du tableau 1 ci-dessous:

**TABLEAU 1**

| **Nom commercial** | **Nom chimique INCI** | **Nom chimique ou description** | **Alcools/acides utilisés** | **Numéro CAS** |
|---|---|---|---|---|
| Castor 22L73 | Hydrogenated Castor Oil Behenyl esters | Huile de ricin est composée à 95% d'acide ricinoléique | Alcool gras en C₂₂ | - |
| Castor 16L64 | Hydrogenated castor oil cetyl esters | | Alcool gras en C₁₆ | - |
| Dynasan 118 | Tristearin | 2,3-di(octadecanoyloxy)propyl octadecanoate, C₅₇H₁₁₀O₆ | Glycérol | 555-43-1 |
| Olive 18L57 | Hydrogenated olive oil stearyl esters | - | Alcool gras en C₁₈ | - |
| Dynasan P60F | Hydrogenated Palm Oil | - | - | 68514-74-9 / 8033-29-2 |
| Huile végétale hydrogénée | | | - | |
| Softisan 154 | | | - | |
| Shellac SSB 63 | Shellac | Gomme laque | - | 9000-59-3 |
| Dynasan 114 | Trimyristin | 1,3-di(tetradecanoyloxy)propan-2-yl tetradecanoate (Glycérol trimyristate),C₄₅H₈₆O₆ | Ester de Glycérol et d'acide tétradecanoique CH₃(CH₂)₁₂COOH | 555-45-3 |
| Lipoxol 6000 | PEG-135 | Polyethylene glycol,HO-(CH₂CH₂O)₁₃₅-H | Polymérisation d'éthane-1,2-diol | 25322-68-3 |
| Cire de Carnauba | Copernicia cerifera cera | - | | 8015-86-9 |
| Cire de Candellila | Candelilla Wax | - | | 8006-44-8 |
| Cire d'abeille | Cera alba | Contient principalement du palmitate de myricyle, C₁₉H₃₁CO₂C₃₁H₆₃ | Ester d'acide palmitique CH₃(CH₂)₁₄COOH et de triacontanol (C₃₀H₆₂O) | 8012-89-3 |
| Imwitor 960 K | Glyceryl Stearate SE | Octadecanoic acid, reaction products with 1,2,3-propanetriol (1:1), neutralized | Ester de Glycérol et d'acide stéarique | 11099-07-3 |
| Imwitor 49 | Glyceryl stearate | Glycéryl monostearate, Octadecanoic acid, - monoester with 1,2,3-propanetriol, C₂₁H₄₂O₄ | Ester de Glycérol et d'acide stéarique | 31566-31-1 |

Avantageusement, la cire est choisie parmi les cires ayant une température de fusion comprise entre 50 et 75°C.

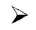 **PRINCIPE ACTIF**

Le principe actif est quant à lui, choisi parmi les liquides et les solides, et peut être liposoluble, lipo-dispersible, non hydrosoluble ou amphiphile ; il est choisi en particulier parmi les molécules volatiles ou non volatiles à température ambiante ou à température d'usage.

On peut, par exemples, citer comme actif : les parfums d'ambiance pour parfumer l'atmosphère ou les parfums destinés à être appliqués sur la peau, les répulsifs d'insectes, les poudres, les colorants, les agents anti-rides, les filtres UV, les vitamines en particulier B3, B8, B12 et B9, les agents hydratants, les agents desquamants, les agents améliorant la fonction barrière, les agents antioxydants, les agents dermodécontractants ou agents dermorelaxants, les agents anti-glycation, les agents stimulant la synthèse des macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules et les agents apaisants.

L'actif peut être présent en une teneur allant de 0,001 à 80% en poids, de préférence de 0,01 à 60 % en poids, et plus préférentiellement de 0,1 à 45% en poids par rapport au poids total de cire.

En outre, pour réaliser le procédé selon l'invention, il faut disposer de deux phases aqueuses : une première phase aqueuse comprenant au moins un tensio-actif hydrosoluble et au moins un colloïde protecteur et une seconde phase aqueuse comprenant au moins un tensio-actif hydrosoluble ou au moins un colloïde protecteur, ou les deux.

### II/ PHASES AQUEUSES:

Afin de prévenir la coalescence des particules liquides de cire au moment de la cristallisation de la cire contenant l'actif encapsulé, un colloïde protecteur est incorporé dans la première phase aqueuse. Il peut aussi être incorporé optionnellement dans la deuxième phase aqueuse ou y être présent seul sans tensioactif soluble.

Le colloïde protecteur peut être choisi parmi la gomme arabique, la gélatine, les dérivés de cellulose, les polyvinylpyrrolidones, ou les alcools polyvinyliques.
La première phase aqueuse peut comprendre jusqu'à 10% en poids de colloïde protecteur par rapport au poids total de ladite phase.
La deuxième phase aqueuse conforme à l'invention peut éventuellement comprendre au moins un colloïde protecteur, tels que cités précédemment dans les proportions indiquées précédemment.

Le tensioactif ou agent de surface est, de façon générale, un composé qui modifie la tension superficielle entre une phase grasse et une phase aqueuse. Parmi les tensioactifs hydrosolubles, conformes à l'invention, on peut citer les tensioactifs anioniques et les tensioactifs non ioniques. Leur nature ne revêt pas, dans le cadre de la présente invention, de caractère véritablement critique.

### ➢ Les tensioactifs anioniques:

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'aminés, sels d'aminoalcools ou sels de magnésium) des composés suivants:
- Les alkylsulfates, les alkyléthersulfates, alkylamidoéthersulfates, alkylarylpolyéthersulfates, monoglycérides sulfates;
- Les alkylsulfonates, alkylphosphates, alkylamidesulfonates, alkylarylsulfonates, alpha -oléfine-sulfonates, paraffine-sulfonates;
- Les alkylsulfosuccinates, les alkyléthersulfosuccinates, les alkylamidesulfosuccinates;
- Les alkylsulfosuccinamates;
- Les alkylsulfoacétates;
- Les alkylétherphosphates;
- Les acylsarcosinates ;
- Les acyliséthionates ; et
- Les N-acyltaurates,
le radical alkyle ou acyle de tous ces différents composés comportant de préférence de 12 à 20 atomes de carbone, et le radical aryle désignant de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone.

On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges.

Les tensioactifs anioniques du type acides ou sels d'éthers carboxyliques polyoxyalkylénés sont en particulier ceux qui répondent à la formule (1) suivante : R1(OC₂H₄)ₙOH₂COOA **(1)**
dans laquelle :
- R1 désigne un groupement alkyle ou alkylaryle,
- n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
- A désigne H, ammonium, Na, K, Li, Mg ou un reste monoéthanolamine ou triéthanolamine. On peut également utiliser des mélanges de composés de formule (1) en particulier des mélanges dans lesquels les groupements R1 sont différents.

Parmi tous ces tensioactifs anioniques, on préfère utiliser plus particulièrement les sels d'alkylsulfates et d'alkyléthersulfates, ainsi que leurs mélanges.

### ➢ Les tensioactifs non ioniques:

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178).

Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant 1 à 5 groupements glycérol et en particulier 1,5 à 4 ; les amines grasses polyéthoxylées ayant de préférence 2 à 30 moles d'oxyde d'éthylène; les esters d'acides gras du sorbitan oxyéthylénés ayant de 2 à 30 moles d'oxyde d'éthylène; les esters d'acides gras du sucrose, les esters d'acides gras de polyéthylèneglycol, les alkylpolyglycosides, les dérivés de N-alkyl glucamine, les oxydes d'aminés tels que les oxydes d'alkyl (C10-C14) aminés ou les oxydes de N-acylaminopropylmorpholine. On notera que les alkylpolyglycosides constituent des tensioactifs non-ioniques rentrant particulièrement bien dans le cadre de la présente invention.

Comme tensio-actifs hydrosolubles particulièrement préférés, on peut citer le Pluronic F68, le Stéareth 21, le Tween 60, les Synperonic.

Le Pluronic F68 est un copolymère polyéthylène polypropylène ayant la masse moléculaire moyenne 8350 et dont le numéro de CAS est 9003-11-6.

Le Tween 60 est un monostéarate de polyoxyéthylène polyoxyprolène sorbitane ayant la masse moléculaire moyenne 1309 et dont le numéro de CAS est 9005-67-8.

La première phase aqueuse comme la deuxième phase aqueuse conforme à l'invention peut comprendre de 0,01 à 20 % en poids d'au moins un tensioactif conforme à l'invention, de préférence de 0,1 à 10 % en poids, par rapport au poids total de ladite phase et plus particulièrement 0,3 à 5% en poids.

D'autres avantages pourront encore apparaître à l'homme du métier à la lecture des exemples ci-dessous, illustrés par les figures annexées, données à titre illustratif. Les exemples 1, 2, 4-10 illustrent la fabrication selon le procédé de l'invention de dispersions de microcapsules à partir de phases aqueuses et/ou de phases grasses de différentes compositions. L'exemple 3 illustre l'état antérieur de la technique et correspond à la reproduction du procédé de US 2006/024248.

### Brève description des figures

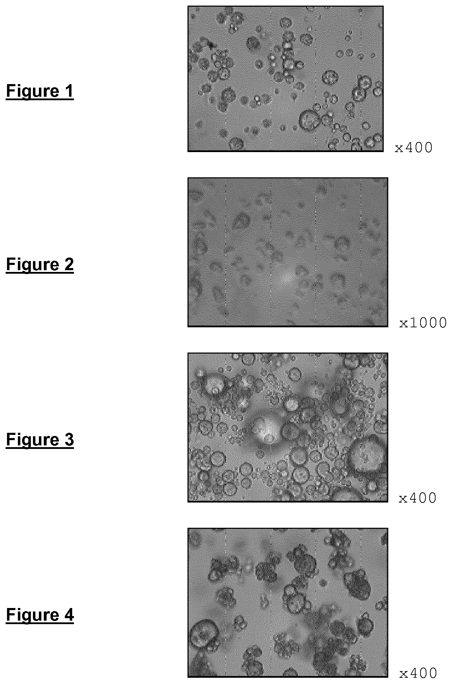

La **figure 1** représente une photographie avec un grossissement x400 d'une dispersion de microcapsules dans l'eau fabriquée selon le procédé de l'invention dont la membrane des microcapsules est en ester d'acide ricinoléique hydrogéné et d'alcools gras primaire et linéaire en C22 ayant une température de fusion de 73°C et dont le principe actif encapsulé est de l'huile d'amande douce.
La **figure 2** représente une photographie avec un grossissement x1000 de la même dispersion que celle de la figure 1
La **figure 3** représente une photographie avec un grossissement x400 d'une dispersion de microcapsules dans l'eau fabriquée selon le procédé de l'invention dont la membrane des microcapsules est en tristéarine et dont le principe actif encapsulé est le lactate de menthyle.
La **figure 4** représente une photographie avec un grossissement x400 d'une dispersion de microcapsules dans l'eau fabriquée selon le procédé de l'invention dont la membranes des microcapsules est en ester d'acide stéarique et d'alcool gras primaire et linéaire en C18 ayant une température de fusion de 57°C et dont le principe actif est le citronellol.

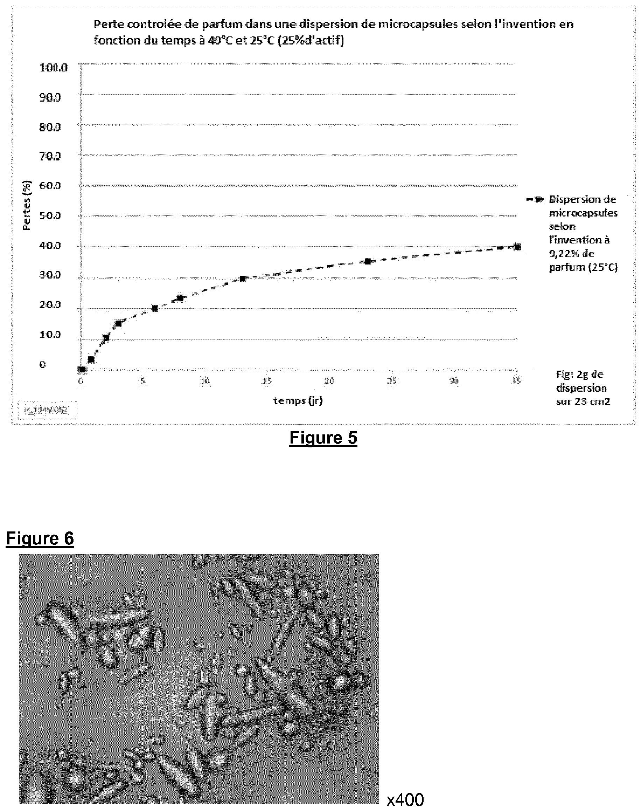

La **figure 5** représente un graphique montrant le relargage « long lasting » d'un parfum encapsulé dans une membrane en tristéarine. La figure 5 montre la perte contrôlée de parfum en fonction du temps à 25°C. L'axe des abscisses correspond au temps auquel a été mesuré le pourcentage de la perte de parfum dans la dispersion de l'exemple 1. L'axe des cordonnées correspond au pourcentage de la perte de parfum.
La **figure 6** représente une photographie avec un grossissement x400 montrant le résultat obtenu dans l'exemple 2 selon le procédé décrit dans l'art antérieur. Il n'y pas formation de microcapsules.

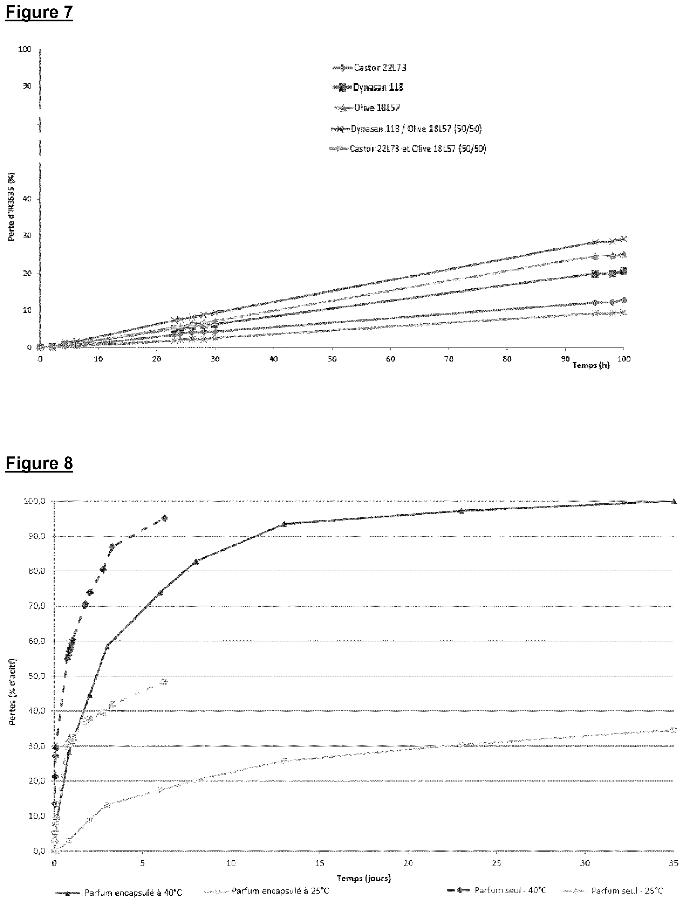

La **figure 7** représente l'influence de la nature de la matrice d'encapsulation sur la vitesse d'évaporation de l'IR3535 à 40°C. Le principe actif IR3535 est encapsulé dans cinq dispersions différentes selon l'invention, chacune contenant une matrice (cire) d'encapsulation différente. L'axe des abscisses correspond au temps auquel a été mesuré le pourcentage de la perte d'actif dans la dispersion.
La **figure 8** représente une comparaison de la vitesse d'évaporation de parfum encapsulé dans une dispersion de microcapsules selon l'invention ou non encapsulé en fonction du temps à 40°C et à 25°C.

Le procédé, objet de l'invention, a les avantages suivants :
- la taille des microcapsules est maîtrisée ;
- les particules peuvent présenter un pic granulométrique fin ;
- ce procédé d'encapsulation est rapide ;
- ce procédé constitue une alternative avec un procédé en batch (pas besoin d'une enceinte particulière) et obtention d'un slurry aqueux.

### EXEMPLES

### Exemple 1 : Fabrication d'une dispersion de microcapsules selon l'invention

La composition de la dispersion est illustrée dans le tableau 2 ci-après.

**Tableau 2**

| **Dispersion** | | |
|---|---|---|
| **Emulsion** | | |
| **1^{ère} Phase aqueuse** | **Phase grasse** | **2^{nde} phase aqueuse** |
| **46,15% d'eau déminéralisée 0,25% de Brij 721 P *** | **27,5% Dynasan 118*** Olive18L57 ***** | Eau déminéralisée 0,5% brij721P* |
| **3,6% de gomme d'acacia **** | 22,5% lactate de menthyle**** | |

| | | |
|---|---|---|
| * Tensio-actif ; ** Colloïde protecteur ; *** Cire ; **** Principe actif. | | |

Le tensioactif Brij 721 P utilisé dans l'exemple 1 est un stéarate de polyoxyéthylène.

Dynasan 118 est la marque de la tristéarine commercialisée.

Le ratio volumique entre la phase grasse et la première phase aqueuse dans l'émulsion est 50 :50.

Le ratio volumique entre l'émulsion et la seconde phase aqueuse dans la dispersion est 70 :30.

Le débit de transfert d'émulsion en « goutte à goutte » est de 13,5ml/min. L'extrait sec de la dispersion obtenue dans l'exemple 1 est de 39,3%. Le diamètre D50 des microcapsules produites dans cet exemple est de 15 µm.

### Matériel utilisé :

| **Réacteur** | **Agitateur** | **Vitesses** |
|---|---|---|
| **Réacteur A pour fabriquer l'Emulsion : Bécher en verre 1L dans un bain Marie à 83°C** | **IKA Eurostar Control® Visc 6000]** | **Fabrication de l'Emulsion à 2800 tr/min** |
| **Réacteur B pour fabriquer la dispersion : Bécher inox 1L dans un bain Marie avec de la glace** | **Heidolph® RZR 1** | **1/ Avant l'ajout de l'émulsion dans la 2nde phase aqueuse : vitesse =1, à savoir 150 tr/mn** |
| | | **2/ Pendant l'ajout de l'émulsion dans la 2nde phase aqueuse : vitesse =6, à savoir 700 tr/mn** |

### Protocole :

La dispersion de microcapsules est fabriquée selon les étapes successives 1-6 suivantes :
1/ Chargement du réacteur A avec les ingrédients de la première phase aqueuse indiqués ci-dessus à une température de 80°C ;
2/ Préparation de la phase grasse en faisant fondre à 80°C la cire et en y ajoutant ensuite le principe actif ;
3/ Ajout de la phase grasse préalablement fondue dans ledit réacteur A, pendant 10 minutes sous agitation dans un agitateur IKA Eurostar Control® Visc 6000 à 2800 tr/min;
4/ Fabrication de la seconde phase aqueuse dans le réacteur B avec les ingrédients indiqués ci-dessus, sous agitation avec un agitateur Heidolph® RZR 1, sous vitesse 150 tr/min;
5/ Ajout au goutte à goutte de l'émulsion présente dans le réacteur A dans le réacteur B sous vitesse élevée 700tr/min ;
6/ Obtention d'une dispersion conforme à l'invention.
Les microcapsules obtenues dans la dispersion de l'exemple 1 sont illustrées par la figure 3.

### Test de perte de parfum dans la dispersion

La perte de parfum encapsulé dans la dispersion ainsi obtenue a été mesurée par perte de masse en évaporation selon une méthode connue de l'homme du métier.

Les microcapsules de l'invention permettent une libération contrôlée du principe actif encapsulé (figures 5 et 8) par rapport à celle du principe actif non encapsulé (figure 8).

### Exemple 2 : Fabrication de trois dispersions de microcapsules illustrées respectivement aux figures 1, 2, 4

Une dispersion de microcapsules en ester d'acide ricinoléique hydrogéné et d'alcools gras primaires et linéaires en C22 a été obtenue selon le protocole décrit ci-dessus.

Les microcapsules obtenues dans cette dispersion sont illustrées par la figure 1 ou la figure 2.

Une dispersion de microcapsules en ester d'acide stéarique et d'alcool gras primaire et linéaire en C18 a été obtenue selon le protocole décrit ci-dessus. Les microcapsules obtenues dans cette dispersion sont illustrées par la figure 4.

### Exemple 3 : Fabrication d'une dispersion de microcapsules selon le procédé de US 2006/024248

Différent du procédé de la présente invention, le procédé selon US 2006/024248 utilise au moins un tensioactif dans la phase grasse. Les compositions de la 1^{ère} et 2^{ème} phases aqueuses et celle de la phase grasse sont illustrées dans le tableau ci-après. La phase grasse n'est pas introduite goutte à goutte dans la phase aqueuse.

La composition de ladite dispersion est illustrée dans le tableau 3 ci-après.

**Tableau 3**

| **Dispersion** | | |
|---|---|---|
| **Emulsion** | | |
| **1^{ère} Phase aqueuse** | **Phase grasse** | **2^{nde} phase aqueuse** |
| **65,9% d'eau déminéralisée** | **20% Huile GV60 ***** | Eau déminéralisée |
| **1% Brij 721 P *** | 10% parfum**** | |
| **2% cire d'acacia **** | 1%Brij72 * | |
| **0,1% Nipastat** | | |

| | | |
|---|---|---|
| * Tensio-actif ; ** Colloïde protecteur ; *** Cire ; **** Principe actif. | | |

Les tensioactifs Brij 721 P et Brij 72 utilisés dans l'exemple 1 sont tous les deux des stéarates de polyoxyéthylène.

L'huile GV 60 est l'huile de palme hydrogénée (température de fusion 60°C)

### Protocole :

1. Préparation de la 1^{ère} phase aqueuse avec les ingrédients indiqués ci-dessus à une température de 70°C sous agitation à 300 rpm pendant 15 minutes.
2. Préparation de la phase grasse avec les ingrédients indiqués ci-dessus à une température de 70°C sous agitation à 300 rpm pendant 15 minutes.
3. Versement de la phase grasse préalablement fondue dans la 1^{ère} phase aqueuse à 70°C.
4. Formation de l'émulsion à 75°C sous agitation à 1100 rpm.
5. Refroidissement de l'émulsion jusqu'à la température ambiante pendant 30 minutes dans un réacteur double enveloppe.
Après le refroidissement, il n'y a pas de microcapsules formées (Figure 6).

### Exemple 4 : Fabrication d'une dispersion de microcapsules selon l'invention

La composition de ladite dispersion est illustrée dans le tableau 4 ci-après.

**Tableau 4**

| Dispersion | | |
|---|---|---|
| Emulsion | | |
| 1^{ère} phase aqueuse | Phase grasse | 2^{nde} phase aqueuse |
| 46,15% eau déminéralisée | | |
| 0,15% Brij 721P * | 34% Dynasan 118 *** | Eau déminéralisée 0,3% Brij 721P * |
| 0,1% Volpo L3 * | 16% IR3535 **** | 0,2% Volpo L3 * |
| 3,6% gomme d'acacia ** | | |

| | | |
|---|---|---|
| * Tensioactif; ** Colloïde protecteur; *** Cire; **** Principe actif | | |

Le ratio volumique entre la phase grasse et la première phase aqueuse dans l'émulsion est 50 :50.

Le ratio volumique entre l'émulsion et la seconde phase aqueuse dans la dispersion est 70 :30.

La phase grasse est ajoutée dans la 1^{ère} phase aqueuse sous agitation à une vitesse de 2800 tr/min. Le débit de transfert d'émulsion en « goutte à goutte » est de 25,6 ml/min. L'émulsion est ajoutée dans la seconde phase aqueuse sous agitation à une vitesse de 1400 tr/min.

L'extrait sec de la dispersion obtenue dans cet exemple est de 36,5%. Le diamètre D50 des microcapsules produites dans cet exemple est de 24,8 µm.

### Exemple 5 : Fabrication d'une dispersion de microcapsules selon l'invention

La composition de ladite dispersion est illustrée dans le tableau 5 ci-après.

**Tableau 5**

| Dispersion | | |
|---|---|---|
| Emulsion | | |
| 1^{ère} phase aqueuse | Phase grasse | 2^{nde} phase aqueuse |
| 46,15% eau déminéralisée | | |
| 0,25% Brij 721P* | 34% Dynasan 118 *** | Eau déminéralisée |
| 0,25% Luvitec K90 * | 16% IR3535 **** | 0,5% Brij 721P * |
| 3,6% gomme d'acacia ** | | |

| | | |
|---|---|---|
| * Tensioactif; ** Colloïde protecteur; *** Cire; **** Principe actif | | |

Le ratio volumique entre la phase grasse et la première phase aqueuse dans l'émulsion est 50 :50. Le ratio volumique entre l'émulsion et la seconde phase aqueuse dans la dispersion est 70 :30.

La phase grasse est ajoutée dans la 1^{ère} phase aqueuse sous agitation à une vitesse de 2700 tr/min. Le débit de transfert d'émulsion en « goutte à goutte » est de 35 ml/min. L'émulsion est ajoutée dans la seconde phase aqueuse sous agitation à une vitesse de 1400 tr/min.

L'extrait sec de la dispersion obtenue dans cet exemple est de 36,6%. Le diamètre D50 des microcapsules produites dans cet exemple est de 43 µm.

### Exemple 6 : Fabrication d'une dispersion de microcapsules selon l'invention

La composition de ladite dispersion est illustrée dans le tableau 6 ci-après.

**Tableau 6**

| Dispersion | | |
|---|---|---|
| Emulsion | | |
| 1^{ère} phase aqueuse | Phase grasse | 2^{nde} phase aqueuse |
| 46,15% eau déminéralisée | 13,75% Dynasan 118 *** | Eau déminéralisée |
| 0,25% Brij 721P * | 13,75% Olive 18L57 *** | 0,5% Brij 721P * |
| 3,6% gomme d'acacia ** | 22,5% IR3535 **** | 7,2% gomme dacacia ** |

| | | |
|---|---|---|
| * Tensioactif; ** Colloïde protecteur; *** Cire; **** Principe actif | | |

Le ratio volumique entre la phase grasse et la première phase aqueuse dans l'émulsion est 50 :50. Le ratio volumique entre l'émulsion et la seconde phase aqueuse dans la dispersion est 65 :35.

La phase grasse est ajoutée dans la 1^{ère} phase aqueuse sous agitation à une vitesse de 1100 tr/min. Le débit de transfert d'émulsion en « goutte à goutte » est de 29,6 ml/min. L'émulsion est ajoutée dans la seconde phase aqueuse sous agitation à une vitesse de 1000 tr/min.

L'extrait sec de la dispersion obtenue dans cet exemple est de 38,9%. Le diamètre D50 des microcapsules produites dans cet exemple est de 34,6 µm.

### Exemple 7 : Fabrication d'une dispersion de microcapsules selon l'invention

La composition de ladite dispersion est illustrée dans le tableau 7 ci-après.

**Tableau 7**

| Dispersion | | |
|---|---|---|
| Emulsion | | |
| 1^{ère} phase aqueuse | Phase grasse | 2^{nde} phase aqueuse |
| 46,15% eau déminéralisée | 42% Dynasan 118 | Eau déminéralisée |
| 0,25% Brij 721P * | *** | 0,5% Brij 721P * |
| 3,6% gomme d'acacia ** | 8% parfum **** | 7,2% gomme d'acacia ** |

| | | |
|---|---|---|
| * Tensioactif; ** Colloïde protecteur; *** Cire; **** Principe actif | | |

Le ratio volumique entre la phase grasse et la première phase aqueuse dans l'émulsion est 50 :50. Le ratio volumique entre l'émulsion et la seconde phase aqueuse dans la dispersion est 70 :30.

La phase grasse est ajoutée dans la 1^{ère} phase aqueuse sous agitation à une vitesse de 1250 tr/min. Le débit de transfert d'émulsion en « goutte à goutte » est de 20,3 ml/min. L'émulsion est ajoutée dans la seconde phase aqueuse sous agitation à une vitesse de 1000 tr/min.

L'extrait sec de la dispersion obtenue dans cet exemple est de 40%. Le diamètre D50 des microcapsules produites dans cet exemple est de 30 µm.

### Exemple 8 : Fabrication d'une dispersion de microcapsules selon l'invention

La composition de ladite dispersion est illustrée dans le tableau 8 ci-après.

**Tableau 8**

| Dispersion | | |
|---|---|---|
| Emulsion | | |
| 1^{ère} phase aqueuse | Phase grasse | 2^{nde} phase aqueuse |
| 32,3% eau déminéralisée | 28% Olive 18L57 *** | Eau déminéralisée |
| 0,18% Brij 721P * | 7% huile d'amande douce **** | 0,15% Brij 721P * |
| 2,52% gomme d'acacia ** | | 2,16% gomme d'acacia ** |

| | | |
|---|---|---|
| * Tensioactif; ** Colloïde protecteur; *** Cire; **** Principe actif | | |

Le ratio volumique entre la phase grasse et la première phase aqueuse dans l'émulsion est 50 :50. Le ratio volumique entre l'émulsion et la seconde phase aqueuse dans la dispersion est 70 :30.

La phase grasse est ajoutée dans la 1^{ère} phase aqueuse sous agitation à une vitesse de 1700 tr/min. Le débit de transfert d'émulsion en « goutte à goutte » est de 25,2 ml/min. L'émulsion est ajoutée dans la seconde phase aqueuse sous agitation à une vitesse de 900 tr/min.

L'extrait sec de la dispersion obtenue dans cet exemple est de 40%. Le diamètre D50 des microcapsules produites dans cet exemple est de 20 µm.

### Exemple 9 : Fabrication d'une dispersion de microcapsules selon l'invention

La composition de ladite dispersion est illustrée dans le tableau 9 ci-après.

**Tableau 9**

| Dispersion | | |
|---|---|---|
| Emulsion | | |
| 1^{ère} phase aqueuse | Phase grasse | 2^{nde} phase aqueuse |
| 46,15% eau déminéralisée | 37,5% Castor 22L73 *** | Eau déminéralisée |
| 0,25% Brij 721P * | 12,5% parfum (dilué à 50% dans du myristate d'isopropyl) **** | Eau 0,5% Brij 721P * |
| 3,6% gomme d'acacia ** | | |

| | | |
|---|---|---|
| * Tensioactif; ** Colloïde protecteur; *** Cire; **** Principe actif | | |

Le ratio volumique entre la phase grasse et la première phase aqueuse dans l'émulsion est 50 :50. Le ratio volumique entre l'émulsion et la seconde phase aqueuse dans la dispersion est 50 :50.

La phase grasse est ajoutée dans la 1^{ère} phase aqueuse sous agitation à une vitesse de 9500 tr/min. Le débit de transfert d'émulsion en « goutte à goutte » est de 12,2 ml/min. L'émulsion est ajoutée dans la seconde phase aqueuse sous agitation à une vitesse de 2800 tr/min.

L'extrait sec de la dispersion obtenue dans cet exemple est de 25%. Le diamètre D50 des microcapsules produites dans cet exemple est de 10 µm.

### Exemple 10 : Fabrication d'une dispersion de microcapsules selon l'invention

La composition de ladite dispersion est illustrée dans le tableau 10 ci-après. La seconde phase aqueuse ne comporte qu'un protecteur colloïde, mais pas de tensioactif.

**Tableau 10**

| Dispersion | | |
|---|---|---|
| Emulsion | | |
| 1^{ère} phase aqueuse | Phase grasse | 2^{nde} phase aqueuse |
| 63% eau déminéralisée | 20% Castor 22L73 *** | Eau déminéralisée 10% gomme d'acacia ** |
| 7% gomme d'acacia ** | 10% huile de thon **** | |

| | | |
|---|---|---|
| ** Colloïde protecteur; *** Cire; **** Principe actif | | |

Le ratio volumique entre la phase grasse et la première phase aqueuse dans l'émulsion est 30 :70. Le ratio volumique entre l'émulsion et la seconde phase aqueuse dans la dispersion est 40 :60.

La phase grasse est ajoutée dans la 1^{ère} phase aqueuse sous agitation à une vitesse de 2500 tr/min. Le débit de transfert d'émulsion en « goutte à goutte » est de 21,4 ml/min. L'émulsion est ajoutée dans la seconde phase aqueuse sous agitation à une vitesse de 1000 tr/min.

L'extrait sec de la dispersion obtenue dans cet exemple est de 21,2%. Le diamètre D50 des microcapsules produites dans cet exemple est de 20 µm.

### Exemple 11 : Influence de la nature de la matrice d'encapsulation sur la vitesse d'évaporation de l'IR3535

Le relargage « long lasting » de l'IR3535 encapsulé est testé, à 40°C, dans cinq dispersions différentes selon l'invention pour lesquelles ont été utilisées cinq différentes matrices d'encapsulation, à savoir la cire dans la phase grasse. La composition respective de ces cinq dispersions est illustrée dans le tableau 11 ci-après.

**Tableau 11**

| Dispersion | | |
|---|---|---|
| Emulsion | | |
| 1^{ère} phase aqueuse | Phase grasse | 2^{nde} phase aqueuse |
| 32,3% eau déminéralisée | 19,25 % de matrice *** | Eau déminéralisée 0,15% Brij 721P * |
| 0,18% Brij 721P * | | 2,16% gomme d'acacia ** |
| 2,52% gomme d'acacia ** | 15,75% IR3535 **** | |

| | | |
|---|---|---|
| * Tensioactif; ** Colloïde protecteur; *** Cire; **** Principe actif | | |

Les matrices utilisées dans ces cinq dispersions sont respectivement :
- Castor 22L73
- Dynasan 118
- Olive 18L57
- Dynasan 118 / Olive 18L57 (50/50)
- Castor 22L73 / Olive 18L57 (50/50).

Bien que la nature de la matrice d'encapsulation puisse influencer la vitesse d'évaporation du principe actif encapsulé, les microcapsules obtenues selon le procédé de l'invention présentent toutes la capacité de relargage « long lasting » (figure 7).

## Revendications

1. Procédé de fabrication d'une dispersion de microcapsules de principe actif, en suspension dans l'eau comprenant les étapes successives suivantes :
I. chauffage d'une phase grasse comprenant au moins (i) une cire, naturelle et/ou synthétique, et au moins un principe actif à encapsuler, à une température réactionnelle supérieure à la température de fusion de ladite cire;
II. Chauffage d'une première phase aqueuse comprenant au moins un tensio-actif hydrosoluble et au moins un colloïde protecteur, à ladite température réactionnelle, sous agitation mécanique ;
III. Incorporation de ladite phase grasse dans ladite première phase aqueuse en maintenant la température réactionnelle, sous agitation mécanique, de manière à former une émulsion huile-dans-eau stable;
IV. Ajout au goutte à goutte de ladite émulsion formée précédemment dans une seconde phase aqueuse comprenant au moins un tensioactif hydrosoluble ou au moins un colloïde protecteur, la température de ladite seconde phase étant inférieure à la température de fusion de ladite cire sous agitation mécanique et conduisant à la formation de microcapsules solides, cristallisées contenant ledit principe actif, lesdites microcapsules étant en suspension dans ladite seconde phase aqueuse, lesdites microcapsules ayant un diamètre compris entre 2 et 80 micromètres.

2. Procédé selon la revendication 1 dans lequel la température réactionnelle est supérieure d'au moins 10°C à la température de fusion de ladite cire.

3. Procédé selon l'une des revendications précédentes dans lequel la vitesse d'agitation est de 700 à 6000 trs/min, avantageusement entre 1000 et 4000 trs/min avec l'agitateur IKA Eurostar Control Vsic 6000.

4. Procédé selon l'une quelconque des revendications précédentes, dans lequel le principe actif, solide ou liquide, est liposoluble, lipo-dispersible, non hydrosoluble, ou amphiphile.

5. Procédé selon l'une quelconque des revendications précédentes, dans lequel le colloïde protecteur est choisi parmi la gomme arabique, la gélatine, les dérivés de cellulose, les alcools polyvinyliques et les polyvinylpyrrolidones.

6. Procédé selon l'une des revendications précédentes, dans lequel le ou les tensioactif(s) de la première phase aqueuse et/ou de la seconde phase aqueuse est ou sont choisi(s) parmi les tensioactifs non ioniques et les tensioactifs anioniques.

7. Procédé selon la revendication précédente, dans lequel le ou les tensioactif(s) de la première phase aqueuse et/ou de la seconde phase aqueuse est ou sont choisi(s) parmi les polyoxyéthylène stearyl éthers, le lauryl sulfonate sodium, les copolymères polyoxyethylene polyoxypropylène et les esters de sorbitan.

8. Procédé selon l'une quelconque des revendications précédentes dans lequel la cire est choisie parmi les cires ayant une température de fusion comprise entre 23 et 85°C, avantageusement comprise entre 50 et 75°C.

9. Utilisation des microcapsules obtenues à l'issue du procédé selon l'une quelconque des revendications précédentes.

10. Utilisation selon la revendication 9 (i) pour fabriquer une composition cosmétique choisie parmi les crèmes, les lotions, les gels et les émulsions, ou (ii) pour fabriquer des tissus ou du papier imprégné(s), ou (iii) pour fabriquer des produits ménagers, phytosanitaires ou agrochimiques.

## Patentansprüche

1. Verfahren zur Herstellung einer im Wasser suspendierten Mikrokapseldispersion mit Wirkstoff, folgende sukzessive Schritte umfassend:
I. Aufheizen einer Fettphase, die mindestens (i) ein natürliches und/oder synthetisches Wachs und mindestens einen zu verkapselnden Wirkstoff umfasst, auf eine Reaktionstemperatur über der Schmelztemperatur des Wachses;
II. Aufheizen einer ersten wässrigen Phase, die mindestens ein wasserlösliches Tensid und mindestens ein Schutzkolloid umfasst, auf die Reaktionstemperatur durch mechanisches Rühren;
III. Einmischung der Fettphase in die erste wässrige Phase durch mechanisches Rühren, wobei die Reaktionstemperatur gehalten wird, sodass eine stabile Öl-in-Wasser Emulsion gebildet wird;
IV.Tröpfchenweises Hinzufügen der vorher gebildeten Emulsion in eine zweite wässrige Phase, die mindestens ein wasserlösliches Tensid oder mindestens ein Schutzkolloid umfasst, wobei die Temperatur der zweiten Phase niedriger als die Schmelztemperatur des Wachses unter mechanischem Rühren ist und zur Bildung fester, kristallisierten Mikrokapseln führt, welche den Wirkstoff beinhalten, wobei die Mikrokapseln in der zweiten wässrigen Phase suspendiert sind, wobei die Mikrokapseln einen Durchmesser zwischen 2 und 80 µm aufweisen.

2. Verfahren nach Anspruch 1, bei welchem die Reaktionstemperatur um mindestens 10°C höher als die Schmelztemperatur des Wachses liegt.

3. Verfahren nach einem der vorhergehenden Ansprüche, bei welchem die Rührgeschwindigkeit 700 bis 6.000 Umdrehungen/Min., vorzugsweise zwischen 1.000 und 4.000 Umdrehungen/Min. mit dem Rührwerk IKA Eurostar Control Vsic 6000 beträgt.

4. Verfahren nach einem der vorhergehenden Ansprüche, bei welchem der Wirkstoff, ob fest oder flüssig, entweder fettlöslich, fettdispergierbar, nicht wasserlöslich oder amphiphile ist.

5. Verfahren nach einem der vorhergehenden Ansprüche, bei welchem das Schutzkolloid aus den Gummi arabicum, Gelatine, Zellulosederivaten, Polyvinylalkoholen und Polyvinylpyrrolidon ausgesucht wird.

6. Verfahren nach einem der vorhergehenden Ansprüche, bei welchem das oder die Tensid(e) der ersten wässrigen Phase und/oder der zweiten wässrigen Phase aus den nichtionogenen Tensiden und den anionischen Tensiden ausgesucht wird bzw. werden.

7. Verfahren nach einem der vorhergehenden Ansprüche, bei welchem das oder die Tensid(e) der ersten wässrigen Phase und/oder der zweiten wässrigen Phase aus den Polyoxypropylenstearoylethern, Natriumlaurylsulfonat, den Kopolymeren Polyoxyethylen Polyoxypropylen und Sorbitanestern ausgesucht wird bzw. werden.

8. Verfahren nach einem der vorhergehenden Ansprüche, bei welchem das Wachs aus den Wachsen mit einer Schmelztemperatur zwischen 23 und 85 °C, vorzugsweise zwischen 50 und 75 °C, ausgesucht wird.

9. Verwendung der mit dem Verfahren nach einem der vorhergehenden Ansprüche gewonnenen Mikrokapseln.

10. Verwendung nach Anspruch 9 (i) zur Herstellung einer kosmetischen Zusammensetzung, welche aus den Cremes, Lotionen, Gels und Emulsionen ausgesucht wird, oder (ii) zur Herstellung imprägnierter Stoffe oder imprägnierten Papiers, oder (iii) zur Herstellung von Haushaltsprodukten, Pflanzenschutzmitteln oder agrochemischen Produkten.

## Claims

1. Method for the production of a dispersion of microcapsules of active ingredient, in suspension in water comprising the following successive steps:
I. Heating an oil phase comprising at least (i) one wax, natural and/or synthetic, and at least one active ingredient to be encapsulated, at a reaction temperature greater than the melting temperature of said wax;
II. Heating a first aqueous phase comprising at least one water-soluble surfactant and at least one protective colloid, at said reaction temperature, under mechanical stirring;
III. Incorporating said oil phase into said first aqueous phase while maintaining the reaction temperature, under mechanical stirring, so as to form a stable oil-in-water emulsion;
IV. Adding dropwise said previously formed emulsion into a second aqueous phase comprising at least one water-soluble surfactant or at least one protective colloid, the temperature of said second phase being less than the melting temperature of said wax under mechanical stirring and leading to the formation of solid, crystallized microcapsules containing said active ingredient, said microcapsules being in suspension in said second aqueous phase,
said microcapsules having a diameter comprised between 2 and 80 micrometres.

2. Method according to claim 1 in which the reaction temperature is greater by at least 10°C than the melting temperature of said wax.

3. Method according to one of the preceding claims in which the speed of stirring is from 700 to 6000 rpm, advantageously between 1,000 and 4,000 rpm with the IKA Eurostar Control Vsic 6000 stirrer.

4. Method according to any one of the preceding claims, in which the active ingredient, solid or liquid, is liposoluble, lipo-dispersible, non water-soluble, or amphiphilic.

5. Method according to any one of the preceding claims, in which the protective colloid is selected from gum arabic, gelatine, cellulose derivatives, polyvinyl alcohols and polyvinylpyrrolidones.

6. Method according to one of the preceding claims, in which the surfactant(s) of the first aqueous phase and/or of the second aqueous phase is or are selected from the non-ionic surfactants and the anionic surfactants.

7. Method according to the preceding claim, in which the surfactant(s) of the first aqueous phase and/or of the second aqueous phase is or are selected from polyoxyethylene stearyl ethers, sodium lauryl sulphonate, polyoxyethylene polyoxypropylene copolymers and sorbitan esters.

8. Method according to any one of the preceding claims in which the wax is selected from the waxes having a melting temperature comprised between 23 and 85°C, advantageously comprised between 50 and 75°C.

9. Use of the microcapsules obtained at the end of the method according to any one of the preceding claims.

10. Use according to claim 9 (i) in order to produce a cosmetic composition selected from creams, lotions, gels and emulsions, or (ii) in order to produce impregnated fabrics or paper, or (iii) in order to produce household, phytosanitary or agrochemical products.
